# EUROPÄISCHE PATENTSCHRIFT

(11) **EP 0 560 087 B1**
(45) Veröffentlichungstag und Bekanntmachung des Hinweises auf die Patenterteilung: **04.09.1996**
(21) Anmeldenummer: 93102426.9
(22) Anmeldetag: 17.02.1993
(51) Int. Cl.: C07F 3/00, C07C 68/08, C07C 31/30, D21H 25/18

(54) **Verfahren zur Stabilisierung ethanolischer Ethylmagnesiumcarbonat-Lösungen**
Method for the stabilization of solutions of ethylmagnesium-carbonate in ethanol
Procédé pour la stabilisation de solutions de carbonate d'éthylmagnésium dans éthanol

(30) Priorität: 12.03.1992 DE 4207883
(43) Veröffentlichungstag der Anmeldung: 15.09.1993
(73) Patentinhaber: HOECHST AKTIENGESELLSCHAFT, 65926 Frankfurt am Main (DE)
(72) Erfinder: Jägers, Erhard, Dr., W-5303 Bornheim 4 (DE); Seidel, Andreas, Dr., W-5000 Köln 41 (DE); Pottkämper, Karen, W-5030 Hürth (DE)

(56) Entgegenhaltungen:
- GB-A- 2 055 886
- US-A- 3 277 002
- US-A- 3 761 411
- US-A- 4 123 446
- US-A- 4 318 963

## Beschreibung

Die Erfindung betrifft ein Verfahren zur Stabilisierung einer Lösung von Ethylmagnesiumcarbonat in Ethanol, die durch Umsetzung einer Suspension von Magnesiumethylat in Ethanol mit Kohlendioxid hergestellt wurde.

Alkylmagnesiumcarbonate sind schwache Basen, die im Lebensmittelsektor und vor allem auf dem Gebiet der Massenentsäuerung von Büchern eingesetzt werden. Dabei bewirken die Alkylmagnesiumcarbonate die Neutralisation der im Papier freigesetzten Schwefelsäure; kleinere Mengen wandeln sich in Magnesiumhydroxid um und verbleiben nach der Behandlung als Puffer im Papier.

Für diesen Zweck wurde bisher in den meisten Fällen Methylmagnesiumcarbonat in einer methanolischen Lösung verwendet. Bei der Neutralisation entsteht aus Methylmagnesiumcarbonat dann Magnesiumsulfat, Kohlendioxid und Methanol.

Nun sind Methanol und damit auch Methylmagnesiumcarbonat toxische Substanzen, bei denen die Gefahr besteht, daß kleine Mengen im Buch verbleiben und somit bei der Benutzung zu Problemen führen.
Als wassergefährdender Stoff bereitet Methanol auch beim eigentlichen Entsäuerungsprozess zusätzlich Schwierigkeiten.

Aus diesen Gründen würde der Einsatz von Ethylmagnesiumcarbonat wesentlich günstiger sein. Lösungen von Ethylmagnesiumcarbonat in Ethanol haben aber leider die Eigenschaft, auch bei relativ kleinen Konzentrationen nicht stabil zu sein. Nach kurzer Zeit kommt es zur Kristallisation des Ethylmagnesiumcarbonats, wodurch die Lösungen unbrauchbar werden.

Die US-A-4,318,963 beschreibt ein Verfahren zur Behandlung von cellulosischen Materialien, bei der eine alkoholische Lösung eines Alkoxymagnesiumcarbonats eingesetzt wird. Hierbei sind Verbindungen mit Methoxy- und Ethoxy-Gruppen bevorzugt. Da die erzeugten Lösungen nicht stabil sind, müssen sie sofort nach ihrer Herstellung verwendet werden.

Es wurde nun überraschend gefunden, daß ethanolische Lösungen von Ethylmagnesiumcarbonat dadurch stabilisiert werden können, daß man ihnen bzw. ihren Ausgangsstoffen in geringem Mengen Wasser zusetzt. Auf diese Art und Weise bleiben auch hochviskose Lösungen mit Magnesiumgehalten von über 5 % stabil.

Die erforderliche Wassermenge bewegt sich im Bereich von 0,2 - 1 Masse%, bevorzugt im Bereich von 0,6 - 0,8 Masse%, bezogen auf die Gesamtlösung. Bei geringeren Werten sind die Lösungen nicht auf Dauer stabil, bei höheren Werten kommt es zu Ausfällerscheinungen.

Die erforderliche Wassermenge wird am günstigsten dem Ethanol bei der Herstellung des Ethylmagnesiumcarbonats zugefügt. Sie kann aber auch der fertigen ethanolischen Lösung zugesetzt werden.

Im einzelnen ist das Verfahren der Erfindung zur Stabilisierung einer Lösung von Ethylmagnesiumcarbonat in Ethanol, die durch Umsetzung einer Suspension von Magnesiumethylat in Ethanol mit Kohlendioxid hergestellt wurde, nunmehr dadurch gekennzeichnet, daß man der Lösung oder ihren Ausgangsstoffen 0,2 bis 1,0 Masse% Wasser zufügt, berechnet auf die Lösung des Ethylmagnesiumcarbonats.

Weiterhin kann das Verfahren der Erfindung wahlweise und bevorzugt dadurch gekennzeichnet sein, daß man
a) bei der Herstellung der Lösung von Ethylmagnesiumcarbonat Ethanol mit dem berechneten Wassergehalt einsetzt;
b) die berechnete Menge Wasser der Suspension von Magnesiumethylat in Ethanol zufügt;
c) die berechnete Menge Wasser der fertigen Lösung von Ethylmagnesiumcarbonat in Ethanol zufügt.

Die Temperatur der Umsetzung kann zwischen 0 und 5o °C, bevorzugt zwischen 2o und 3o °C liegen.

Die Konzentration der zu stabilisierenden Lösung von Ethylmagnesiumcarbonat in Ethanol kann zwischen 0,5 und 1o Masse%, vorzugsweise zwischen 4 und 6 Masse%, berechnet auf Magnesium, liegen.

### Beispiel 1 (Vergleichsversuch)

In einen mit Rührer, Gaseinleitungsrohr, Thermometer und Rückflußkühler bestückten 1-Liter Kolben werden 337,2 g wasserfreies Ethanol vorgelegt. Anschließend werden 117,0 g pulverförmiges Magnesiumethylat dazugegeben.
Nach 10 Minuten wird die Suspension bei Raumtemperatur mit CO₂ bis zur Sättigung begast. Die Reaktion verläuft schwach exotherm. Zur Vervollständigung der Reaktion wird noch 30 Minuten nachgerührt. Das Produkt wird in einem geschlossenen Behälter mit Stickstoff überlagert.
Nach etwa 24 h besteht die Lösung aus einer kristallinen Masse von ausgefallenem Ethylmagnesiumcarbonat.

### Beispiel 2

In einem mit Rührer, Gaseinleitungsrohr, Thermometer und Rückflußkühler bestückten 1-Liter Kolben werden 337,2 g absolutes Ethanol mit 3,6 g Wasser versetzt. Anschließend werden 117,0 g pulverförmiges Magnesiumethylat dazugegeben.

Nach 10 Minuten wird die Suspension bei Raumtemperatur mit CO₂ bis zur Sättigung begast. Die Reaktion verläuft schwach exotherm. Zur Vervollständigung der Reaktion wird noch 30 Minuten nachgerührt. Das Produkt wird in einem geschlossenen Behälter mit Stickstoff überlagert.
Die so hergestellte Lösung ist auch nach Wochen noch klar und frei von ausgefallenen kristallinen Produkten.

### Beispiel 3

In einen mit Rührer, Gaseinleitungsrohr, Thermometer und Rückflußkühler bestückten 1-Liter Kolben werden 337,2 g wasserfreies Ethanol vorgelegt. Anschließend werden 117,0 g pulverförmiges Magnesiumethylat dazugegeben.
Nach 10 Minuten wird die Suspension bei Raumtemperatur mit CO₂ bis zur Sättigung begast. Die Reaktion verläuft schwach exotherm. Zur Vervollständigung der Reaktion wird noch 30 Minuten nachgerührt. Danach werden der Lösung 3,6 g Wasser zugesetzt. Das Produkt wird in einem geschlossenen Behälter mit Stickstoff überlagert.
Die so hergestellte Lösung ist auch nach Wochen noch klar und frei von ausgefallenen kristallinen Produkten.

## Patentansprüche

1. Verfahren zur Stabilisierung einer Lösung von Ethylmagnesiumcarbonat in Ethanol, die durch Umsetzung einer Suspension von Magnesiumethylat in Ethanol mit Kohlendioxid hergestellt wurde, dadurch gekennzeichnet, daß man der Lösung oder ihren Ausgangsstoffen 0,2 bis 1,0 Masse% Wasser zufügt, berechnet auf die Lösung des Ethylmagnesiumcarbonats.

2. Verfahren nach Anspruch 1, dadurch gekennzeichnet, daß man bei der Herstellung der Lösung von Ethylmagnesiumcarbonat Ethanol mit dem berechneten Wassergehalt einsetzt.

3. Verfahren nach Anspruch 1, dadurch gekennzeichnet, daß man die berechnete Menge Wasser der Suspension von Magnesiumethylat in Ethanol zufügt.

4. Verfahren nach Anspruch 1, dadurch gekennzeichnet, daß man die berechnete Menge Wasser der fertigen Lösung von Ethylmagnesiumcarbonat in Ethanol zufügt.

## Claims

1. A process for the stabilization of a solution of ethylmagnesium carbonate in ethanol, which has been prepared by reaction of a suspension of magnesium ethylate in ethanol with carbon dioxide, which comprises adding 0.2 to 1.0% by weight of water, based on the ethylmagnesium carbonate solution, to the solution or its starting materials.

2. The process as claimed in claim 1, wherein ethanol with the calculated water content is used in the preparation of the ethylmagnesium carbonate solution.

3. The process as claimed in claim 1, wherein the calculated amount of water is added to the suspension of magnesium ethylate in ethanol.

4. The process as claimed in claim 1, wherein the calculated amount of water is added to the finished solution of ethylmagnesium carbonate in ethanol.

## Revendications

1. Procédé pour stabiliser une solution de carbonate d'éthylmagnésium dans l'éthanol qui a été préparée par réaction d'une suspension d'éthylate de magnésium dans l'éthanol avec le dioxyde de carbone, caractérisé en ce que l'on ajoute à la solution ou aux composants de départ de 0,2 à 1,0% en poids d'eau par rapport à la solution du carbonate d'éthylmagnésium.

2. Procédé selon revendication 1, caractérisé en ce que, à la préparation de la solution de carbonate d'éthylmagnésium, on utilise un éthanol contenant la quantité d'eau calculée.

3. Procédé selon revendication 1, caractérisé en ce que l'on ajoute la quantité d'eau calculée à la suspension d'éthylate de magnésium dans l'éthanol.

4. Procédé selon revendication 1, caractérisé en ce que l'on ajoute la quantité calculée à la solution finie du carbonate d'éthylmagnésium dans l'éthanol.
